# EUROPEAN PATENT SPECIFICATION

(11) **EP 1 032 406 B1**
(45) Date of publication and mention of the grant of the patent: **24.08.2005**
(21) Application number: 98954635.3
(22) Date of filing: 18.11.1998
(51) Int. Cl.: A61K 33/06, A61K 31/20, A61P 3/00

(54) **REDUCING BLOOD CHOLESTEROL BY IN VIVO PRECIPITATION OF BILE CONSTITUENTS WITH GROUP II OR -III METAL SUBSTANCES**
BLUTCHOLESTERINSENKUNG DURCH DIE IN VIVO FÄLLUNG VON GALLENBESTANDTEILEN MIT GRUPPE II ODER -III -METALLVERBINDUNGEN
DIMINUTION DU CHOLESTEROL SANGUIN PAR PRECIPITATION IN VIVO DE CONSTITUANTS BILIAIRES A L'AIDE DE SUBSTANCES METALLIQUES DES GROUPES II ET III

(30) Priority: 26.11.1997 GB 9724840
(43) Date of publication of application: 06.09.2000
(73) Proprietor: KAPPA PHARMACEUTICALS LIMITED, Reading RG8 0EN (GB)
(72) Inventor: WILLIAMS, Alun, Roy, Swansea SA9 1EX (GB)
(74) Representative: Robertson, James Alexander
(86) International application number: PCT/GB1998/003475
(87) International publication number: WO 1999/026638

(56) References cited:
- EP-A- 0 087 864
- WO-A-95/05752
- US-A- 4 027 009
- TOUSSAINT ET AL: "Bile Flow and Biliary Blood Cholesterol Levels in Hypercholesteremic Rats After 10 Weeks Treatment with Calcium and Magnesium Sulfate (CMS) Water" BER. BUNDESFORSCHUNGSANST. ERNAERH, vol. BFE-R-93-01, no. 2, 1993, pages 235-238, XP002100565
- TOUSSAINT ET AL: "Absorption and Elimination of Calcium and Magnesium in Normal and Hypercholesteremic Rats During 10-Week Treatment with Calcium and Magnesium Sulphate (CMS) Water" BIOAVAILABILITY,1993, pages 231-234, XP002101029
- TOUSSAINT ET AL: "Effect of Water Containing Calcium and Magnesium Sulfates on the Elimination of Cholesterol in the Rat" ARCHIVES INTERNATIONALES DE PHYSIOLOGIE ET DE BIOCHIMIE, vol. 96, no. 2, June 1988, pages 89-100, XP002100566
- Medline abstract 97007643: Kawasaki et al: "A 13-Week Toxicity Study of Simultaneous Administration of Cochineal and Aluminum-Potassium-Sulfate in Rats" XP002100568 & KAWASAKI ET AL: EISEI SHIKENJO HOKOKU, vol. 112, 1994, pages 48-56,
- YAMAGUCHI ET AL: "Effects of Various Metals on Hepatic Bile Calcium Excretion in Rats: The Stimulatory Effect of Zinc is Mediated Through Acetylcholine Action" RES. EXP. MED., vol. 190, no. 2, 1990, pages 145-151, XP002100567
- O'NEIL M.J. ET AL: 'The Merck Index, 12th Edition, entry 373', 1996, MERCK AND CO, INC., WHITEHOUSE STATION, NJ
- "The Merck Index, 12th Edition" 1996, MERCK & CO. INC. , WHITEHOUTES STATION , NJ * page 63, paragraph 373 *

## Description

The present invention concerns the use of precipitors of bile constituents, particularly substances which provide Al³⁺ ions when in aqueous solution, to cause a reduction in body cholesterol levels, together with the use of same in the manufacture of medicaments for causing a reduction in body cholesterol levels and methods of manufacture of same.

High blood cholesterol levels are widely viewed as being undesirable and so it is desirable to effect a reduction in body cholesterol levels. It has previously been found (WO 95/05752) that ions of groups II and III metals may be used to effect a reduction in the absorption of fatty acids. However, it has not been previously suggested that bile constituents - i.e. cholic acid derivatives, their salts and esters - could be precipitated to effect a reduction in blood cholesterol levels.

It is known in the art (Toussaint *et al*., 1993, Bioavailability '93,235-238) to use Calcium Magnesium Sulphate (CMS) water to reduce blood cholesterol levels. Kawasaki, Y. *et al*. (1994, Eisei Shikenjo Hokoku, 112: 48-56: PubMed ID 8854902) show the administration of the highly soluble aluminum potassium sulfate to rats to cause a decrease in body weight gains. However, this does provide aluminium in a highly soluble form. US 4027009 discloses polymers which act as bile acid binding agents, but does not disclose or suggest the compositions of the present invention.

According to the present invention, there is provided the use of a substance which causes the precipitation of a bile constituent in the manufacture of a medicament for reducing body cholesterol levels.

The substance is a substance which provides ions of aluminium when in aqueous solution, particularly in the conditions prevalent in the gut. The solubility product of the substance in the form in which it is administered (to a patient) is greater than that of the ions of the substance plus bile constituent so that the bile constituent is precipitated out. Bile primarily comprises cholic acid derivatives, their salt and esters and so the bile constituent may be a derivative of cholic acid, or a salt or ester thereof.

Bile is secreted by the liver and emulsifies fats in the duodenum prior to and as an aid to their digestion. After cleavage of fatty acids, bile salts and bile acids provide the essential component for the formation of micelles which are absorbed into the body, the bile acid and salts returning to the liver for re-circulation. Each bile acid or salt is discharged into the gut about 20 times each day, particularly after the consumption of food. The precipitation of bile constituents prevents their re-circulation and the liver replaces the lost constituents by using the circulating cholesterol (from which all bile acids, bile salt and bile esters are synthesised) and thus body cholesterol levels are decreased.

The substance is to be provided in a non-toxic dosage. Appropriate dosages may be readily determined using a simple dose-response assay.

The substance may be provided in an active form or it may become active as a result of the chemical environment existing within the gastrointestinal system. By "active" is meant that the solubility product of the substance is greater than that of the substance and bile constituent so that the bile constituent forms a salt with, or is precipitated by, the substance. Dietary ingredients having a lower solubility product than the bile constituent salt should, of course, be avoided since they could cause displacement of the bile constituent from the ion and thus release bile constituents which may become available for absorption. Useful aluminium salts are the aluminium salts of soaps such as aluminium stearate and aluminium palmitate. These are highly insoluble in aqueous solution and so will not readily release the aluminium as ions unless the aluminium is able to immediately form an even more insoluble salt, which it does with bile constituents.

The substance (i.e. in the form of a medicament) may be administered immediately after consumption of a meal or, for example, within 2 hours of consumption of a meal.

Also provided, according to the present invention, is a method of manufacture of a medicament for reducing body cholesterol levels comprising the use of an aluminium salt of a soap which causes the precipitation of a bile constituent.

Also provided, according to the present invention, is a treatment for reducing body cholesterol levels comprising administering to a patient an aluminium salt of a soap which causes the precipitation of a bile constituent.

## Claims

1. The use of an aluminium salt which is highly insoluble in aqueous solution but less insoluble than the aluminium salts of a bile constituent, and which causes precipitation of the bile constituent, in the manufacture of a medicament for reducing body cholesterol levels, the aluminium salt being the aluminium salt of a soap.

2. The use of an aluminium salt according to claim 1, the aluminium salt being aluminium stearate or aluminium palmitate.

3. The use of an aluminium salt according to either one of the preceding claims, the aluminium salt being provided in a non-toxic dose.

4. The use of an aluminium salt according to any one of the preceding claims, the medicament being taken immediately after consumption of a meal or within 2 hours of consumption of a meal.

## Patentansprüche

1. Verwendung eines Aluminiumsalzes, das in wässriger Lösung hochgradig unlöslich, aber weniger unlöslich als die Aluminiumsalze eines Gallenbestandteils ist und welches die Ausfällung des Gallenbestaridteilz herbeiführt, für die Herstellung eines Arzneimittels zur Reduzierung des Cholesterol-Niveaus, wobei das Aluminiumsalz das Aluminiumsalz einer Seife ist.

2. Verwendung eines Aluminiumsalzes nach Anspruch 1, **dadurch gekennzeichnet, dass** das Aluminiumsalz Aluminiumstearat oder Aluminiumpalmitat ist.

3. Verwendung eines Aluminiumsalzes nach einem der vorhergehenden Anspräche, **dadurch gekennzeichnet, dass** das Aluminiumsalz in einer nichttoxischen Dosis bereitgestellt wird.

4. Verwendung eines Aluminiumsalzes nach einem der vorhergehenden Ansprüche, **dadurch gekennzeichnet, dass** das Arzneimittel unmittelbar nach Verzehr einer Mahlzeit, oder innerhalb von zwei Stunden nach Verzehr einer Mahlzeit eingenommen wird.

## Revendications

1. Utilisation d'un sel d'aluminium qui est très insoluble en solution aqueuse mais molns insoluble que les sels d'aluminium d'un constituant de la bile, et qui provoque la précipitation du constituant de la bile, dans la production d'un médicament pour réduire les concentrations corporelles de cholestérol, le sel d'aluminium étant le sel d'aluminium d'un savon.

2. Utilisation d'un sel d'aluminium selon la revendication 1, le sel d'aluminium étant le stéarate d'aluminium ou le palmitate d'aluminium.

3. Utilisation d'un sel d'aluminium selon l'une quelconque des revendications précédentes, le sel d'aluminium étant apporté en une dose non toxique.

4. Utilisation d'un sel d'aluminium selon l'une quelconque des revendications précédentes, le médicament étant pris Immédiatement après la consommation d'un repas ou dans les 2 heures de la consommation d'un repas.
